# EUROPÄISCHE PATENTSCHRIFT

(11) **EP 2 585 590 B1**
(45) Veröffentlichungstag und Bekanntmachung des Hinweises auf die Patenterteilung: **24.01.2018**
(21) Anmeldenummer: 11726784.9
(22) Anmeldetag: 21.06.2011
(51) Int. Cl.: C12N 9/52, C07K 14/28, C12R 1/63

(54) **NEUE PROTEASEN UND DIESE ENTHALTENDE MITTEL**
NOVEL PROTEASES AND COMPOSITIONS COMPRISING THEM
NOUVELLES PROTÉASES ET AGENTS LES CONTENANT

(30) Priorität: 28.06.2010 DE 102010030609
(43) Veröffentlichungstag der Anmeldung: 01.05.2013
(73) Patentinhaber: Henkel AG & Co. KGaA, 40589 Düsseldorf (DE)
(72) Erfinder: SIEGERT, Petra, 42781 Haan (DE); MERKEL, Marion, 51145 Köln (DE); KLUIN, Cornelia, 40593 Düsseldorf (DE); MAURER, Karl-Heinz, 40699 Erkrath (DE); O'CONNELL, Timothy, 40545 Düsseldorf (DE); WIELAND, Susanne, 41541 Zons/Dormagen (DE); HELLMUTH, Hendrik, 40237 Düsseldorf (DE)
(86) Internationale Anmeldenummer: PCT/EP2011/060306
(87) Internationale Veröffentlichungsnummer: WO 2012/000829

(56) Entgegenhaltungen:
- WO-A1-00/61769
- WO-A1-92/21760
- WO-A1-2010/060822
- DATABASE UniProt [Online] 24. November 2009 (2009-11-24), "SubName: Full=Alkaline serine protease;", XP000002657359, gefunden im EBI accession no. UNIPROT:C9P5F1 Database accession no. C9P5F1
- DATABASE Geneseq [Online] 16. Juli 2002 (2002-07-16), "Alkaline protease-related protein #2.", XP000002657360, gefunden im EBI accession no. GSP:AAM48430 Database accession no. AAM48430
- KWON Y T ET AL: "Cloning and characterization of the gene encoding an extracellular alkaline serine protease from Vibrio metschnikovii strain RH530", GENE, ELSEVIER, AMSTERDAM, NL, Bd. 152, Nr. 1, 1. Januar 1995 (1995-01-01), Seiten 59-63, XP004042588, ISSN: 0378-1119, DOI: 10.1016/0378-1119(94)00648-C
- KEMEL JELLOULI ET AL: "Molecular and biochemical characterization of an extracellular serine-protease from Vibrio metschnikovii J1", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, Bd. 36, Nr. 7, 24. April 2009 (2009-04-24) , Seiten 939-948, XP019665634, ISSN: 1476-5535

### SEQUENCE LISTING

<110> Henkel AG & Co. KGaA
<120> Neue Proteasen und sie enthaltende Mittel
<130> H 09057 PCT
<150> DE 102010030609
   <151> 2010-06-28
<160> 8
<170> PatentIn version 3.3
<210> 1
   <211> 1269
   <212> DNA
   <213> Vibrio metschnikovii
<220>
   <221> CDS
   <222> (1)..(1266)
<220>
   <221> mat_peptide
   <222> (418)..(1266)
<400> 1
   atg ttg aag aaa aat  gtc aaa cgt aca gta  ctg gct gga tta tta        45
   Met Leu Lys Lys Asn  Val Lys Arg Thr Val  Leu Ala Gly Leu Leu
                   -135                 -130                 -125
   ttg cca aca tca atc  tcc ctg gca gtt gca  tct cag ctc aag gat        90
   Leu Pro Thr Ser Ile  Ser Leu Ala Val Ala  Ser Gln Leu Lys Asp
                   -120                 -115                 -110
   caa gaa gta ccg agt  ttt aca cct tct gtt  gcg att gaa aat cat caa   138
   Gln Glu Val Pro Ser  Phe Thr Pro Ser Val  Ala Ile Glu Asn His Gln
                   -105                 -100                 -95
   gca gaa cag cgt tat ttt gtc acc tat gta cct ggt gca acc agc ggg     186
   Ala Glu Gln Arg Tyr Phe Val Thr Tyr Val Pro Gly Ala Thr Ser Gly
               -90                 -85                 -80
   cct atg cgg atg agt caa aac ggc tta acg gaa act gac ttc tca ctg     234
   Pro Met Arg Met Ser Gln Asn Gly Leu Thr Glu Thr Asp Phe Ser Leu
           -75                 -70                 -65
   caa aaa gcc gcc gat ata ttg agc acg gag caa gta acc gtg atc aat     282
   Gln Lys Ala Ala Asp Ile Leu Ser Thr Glu Gln Val Thr Val Ile Asn
       -60                 -55                 -50
   cac ctc gaa tca tta cat act tct gtg gtt gaa atg acg cca agt caa     330
   His Leu Glu Ser Leu His Thr Ser Val Val Glu Met Thr Pro Ser Gln
   -45                 -40                 -35                 -30
   gcg aag caa ctg ctc gat aat cct gat gtg gcg atg atc gaa gtt gat     378
   Ala Lys Gln Leu Leu Asp Asn Pro Asp Val Ala Met Ile Glu Val Asp
                   -25                 -20                 -15
   cca ata cgt tac tta ttc gat gct gag gtt gaa cct tac gca cag caa     426
   Pro Ile Arg Tyr Leu Phe Asp Ala Glu Val Glu Pro Tyr Ala Gln Gln
               -10                 -5                  -1  1
   acg cca tac gga atc cgt atg gta caa gcc gat caa ctc tct gac gtc       474
   Thr Pro Tyr Gly Ile Arg Met Val Gln Ala Asp Gln Leu Ser Asp Val
       5                   10                  15
   tat gca gct aat cgt aaa gtt tgc gtc atc gac tcg ggg tat ctt cgc       522
   Tyr Ala Ala Asn Arg Lys Val Cys Val Ile Asp Ser Gly Tyr Leu Arg
   20                  25                  30                  35
   aac cat att gat cta ccg agc gct gga gtc aca ggc agc act ttc tct       570
   Asn His Ile Asp Leu Pro Ser Ala Gly Val Thr Gly Ser Thr Phe Ser
                   40                  45                  50
   ggc cat ggc tca tgg ttc act gat ggt aat ggt cat gga act cat gtc       618
   Gly His Gly Ser Trp Phe Thr Asp Gly Asn Gly His Gly Thr His Val
               55                  60                  65
   gcg gga acg att gtc gct ctg gat aat aat gtg ggg gtt gtc ggg gtc       666
   Ala Gly Thr Ile Val Ala Leu Asp Asn Asn Val Gly Val Val Gly Val
           70                  75                  80
   tta cca tct ggc tta gtc ggc cta cac aac gta aaa att ttt aac gat       714
   Leu Pro Ser Gly Leu Val Gly Leu His Asn Val Lys Ile Phe Asn Asp
       85                  90                  95
   tcc ggt gtc tgg act cgt gct tcg gac ttg att caa gct atc caa tct       762
   Ser Gly Val Trp Thr Arg Ala Ser Asp Leu Ile Gln Ala Ile Gln Ser
   100                 105                 110                 115
   tgt caa agc gca ggc agt cat gtg gtg aat atg agt ttg ggt ggt agc       810
   Cys Gln Ser Ala Gly Ser His Val Val Asn Met Ser Leu Gly Gly Ser
                   120                 125                 130
   caa ggc agc gta acc gaa caa aac gca atg cgt aac ttt tac caa caa       858
   Gln Gly Ser Val Thr Glu Gln Asn Ala Met Arg Asn Phe Tyr Gln Gln
               135                 140                 145
   ggg atg cta tta gtc gct gct gcg ggt aac tca ggg aat agc agc ttc       906
   Gly Met Leu Leu Val Ala Ala Ala Gly Asn Ser Gly Asn Ser Ser Phe
           150                 155                 160
   tca tac cct gcg tct tat gat gcg gtg gtc tct gtt gcc gcc gtt aat       954
   Ser Tyr Pro Ala Ser Tyr Asp Ala Val Val Ser Val Ala Ala Val Asn
       165                 170                 175
   tcc agt ggc aat cga gct aac ttt tca cag gcc aat tca caa gtt gaa      1002
   Ser Ser Gly Asn Arg Ala Asn Phe Ser Gln Ala Asn Ser Gln Val Glu
   180                 185                 190                 195
   ctc tcg gca cca gga gtt ggg gta cta tca act ggt aat aat ggc ggc      1050
   Leu Ser Ala Pro Gly Val Gly Val Leu Ser Thr Gly Asn Asn Gly Gly
                   200                 205                 210
   tat tta agt tat agt ggt act tct atg gca tca cct cac gtt gcg ggt      1098
   Tyr Leu Ser Tyr Ser Gly Thr Ser Met Ala Ser Pro His Val Ala Gly
               215                 220                 225
   gtc gca gcg ctg gtt tgg agc cac ttc cca caa tgt cga cca gag cga      1146
   Val Ala Ala Leu Val Trp Ser His Phe Pro Gln Cys Arg Pro Glu Arg
           230                 235                 240
   atc cgt caa tcc ttg aat cag act gct tta gat cga ggg gct gcg ggt      1194
   Ile Arg Gln Ser Leu Asn Gln Thr Ala Leu Asp Arg Gly Ala Ala Gly
       245                 250                 255
   aga gat aat ctc tac ggt tgg gga att gtt caa gcg aga gcc gcc tat      1242
   Arg Asp Asn Leu Tyr Gly Trp Gly Ile Val Gln Ala Arg Ala Ala Tyr
   260                 265                 270                 275
   aac tgg tta tct cgc aat ggc tgt taa                                  1269
   Asn Trp Leu Ser Arg Asn Gly Cys
                   280
<210> 2
   <211> 422
   <212> PRT
   <213> Vibrio metschnikovii
<400> 2
   Met Leu Lys Lys Asn  Val Lys Arg Thr Val  Leu Ala Gly Leu Leu
                   -135                 -130                 -125
   Leu Pro Thr Ser Ile  Ser Leu Ala Val Ala  Ser Gln Leu Lys Asp
                   -120                 -115                 -110
   Gln Glu Val Pro Ser  Phe Thr Pro Ser Val  Ala Ile Glu Asn His Gln
                   -105                 -100                 -95
   Ala Glu Gln Arg Tyr Phe Val Thr Tyr Val Pro Gly Ala Thr Ser Gly
               -90                 -85                 -80
   Pro Met Arg Met Ser Gln Asn Gly Leu Thr Glu Thr Asp Phe Ser Leu
           -75                 -70                 -65
   Gln Lys Ala Ala Asp Ile Leu Ser Thr Glu Gln Val Thr Val Ile Asn
       -60                 -55                 -50
   His Leu Glu Ser Leu His Thr Ser Val Val Glu Met Thr Pro Ser Gln
   -45                 -40                 -35                 -30
   Ala Lys Gln Leu Leu Asp Asn Pro Asp Val Ala Met Ile Glu Val Asp
                   -25                 -20                 -15
   Pro Ile Arg Tyr Leu Phe Asp Ala Glu Val Glu Pro Tyr Ala Gln Gln
               -10                 -5              -1  1
   Thr Pro Tyr Gly Ile Arg Met Val Gln Ala Asp Gln Leu Ser Asp Val
       5                   10                  15
   Tyr Ala Ala Asn Arg Lys Val Cys Val Ile Asp Ser Gly Tyr Leu Arg
   20                  25                  30                  35
   Asn His Ile Asp Leu Pro Ser Ala Gly Val Thr Gly Ser Thr Phe Ser
                   40                  45                  50
   Gly His Gly Ser Trp Phe Thr Asp Gly Asn Gly His Gly Thr His Val
                   55                  60                  65
   Ala Gly Thr Ile Val Ala Leu Asp Asn Asn Val Gly Val Val Gly Val
           70                  75                  80
   Leu Pro Ser Gly Leu Val Gly Leu His Asn Val Lys Ile Phe Asn Asp
       85                  90                  95
   Ser Gly Val Trp Thr Arg Ala Ser Asp Leu Ile Gln Ala Ile Gln Ser
   100                 105                 110                 115
   Cys Gln Ser Ala Gly Ser His Val Val Asn Met Ser Leu Gly Gly Ser
                   120                 125                 130
   Gln Gly Ser Val Thr Glu Gln Asn Ala Met Arg Asn Phe Tyr Gln Gln
               135                 140                 145
   Gly Met Leu Leu Val Ala Ala Ala Gly Asn Ser Gly Asn Ser Ser Phe
           150                 155                 160
   Ser Tyr Pro Ala Ser Tyr Asp Ala Val Val Ser Val Ala Ala Val Asn
       165                 170                 175
   Ser Ser Gly Asn Arg Ala Asn Phe Ser Gln Ala Asn Ser Gln Val Glu
   180                 185                 190                 195
   Leu Ser Ala Pro Gly Val Gly Val Leu Ser Thr Gly Asn Asn Gly Gly
                   200                 205                 210
   Tyr Leu Ser Tyr Ser Gly Thr Ser Met Ala Ser Pro His Val Ala Gly
               215                 220                 225
   Val Ala Ala Leu Val Trp Ser His Phe Pro Gln Cys Arg Pro Glu Arg
           230                 235                 240
   Ile Arg Gln Ser Leu Asn Gln Thr Ala Leu Asp Arg Gly Ala Ala Gly
       245                 250                 255
   Arg Asp Asn Leu Tyr Gly Trp Gly Ile Val Gln Ala Arg Ala Ala Tyr
   260                 265                 270                 275
   Asn Trp Leu Ser Arg Asn Gly Cys
                   280
<210> 3
   <211> 283
   <212> PRT
   <213> Vibrio metschnikovii
<400> 3
   Ala Gln Gln Thr Pro Tyr Gly Ile Arg Met Val Gln Ala Asp Gln Leu
   1               5                   10                  15
   Ser Asp Val Tyr Ala Ala Asn Arg Lys Val Cys Val Ile Asp Ser Gly
               20                  25                  30
   Tyr Leu Arg Asn His Ile Asp Leu Pro Ser Ala Gly Val Thr Gly Ser
           35                  40                  45
   Thr Phe Ser Gly His Gly Ser Trp Phe Thr Asp Gly Asn Gly His Gly
       50                  55                  60
   Thr His Val Ala Gly Thr Ile Val Ala Leu Asp Asn Asn Val Gly Val
   65                  70                  75                  80
   Val Gly Val Leu Pro Ser Gly Leu Val Gly Leu His Asn Val Lys Ile
                   85                  90                  95
   Phe Asn Asp Ser Gly Val Trp Thr Arg Ala Ser Asp Leu Ile Gln Ala
               100                 105                 110
   Ile Gln Ser Cys Gln Ser Ala Gly Ser His Val Val Asn Met Ser Leu
           115                 120                 125
   Gly Gly Ser Gln Gly Ser Val Thr Glu Gln Asn Ala Met Arg Asn Phe
       130                 135                 140
   Tyr Gln Gln Gly Met Leu Leu Val Ala Ala Ala Gly Asn Ser Gly Asn
   145                 150                 155                 160
   Ser Ser Phe Ser Tyr Pro Ala Ser Tyr Asp Ala Val Val Ser Val Ala
                   165                 170                 175
   Ala Val Asn Ser Ser Gly Asn Arg Ala Asn Phe Ser Gln Ala Asn Ser
               180                 185                 190
   Gln Val Glu Leu Ser Ala Pro Gly Val Gly Val Leu Ser Thr Gly Asn
           195                 200                 205
   Asn Gly Gly Tyr Leu Ser Tyr Ser Gly Thr Ser Met Ala Ser Pro His
       210                 215                 220
   Val Ala Gly Val Ala Ala Leu Val Trp Ser His Phe Pro Gln Cys Arg
   225                 230                 235                 240
   Pro Glu Arg Ile Arg Gln Ser Leu Asn Gln Thr Ala Leu Asp Arg Gly
                   245                 250                 255
   Ala Ala Gly Arg Asp Asn Leu Tyr Gly Trp Gly Ile Val Gln Ala Arg
               260                 265                 270
   Ala Ala Tyr Asn Trp Leu Ser Arg Asn Gly Cys
           275                 280
<210> 4
   <211> 269
   <212> PRT
   <213> Bacillus lentus
<400> 4
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 5
   <211> 283
   <212> PRT
   <213> Vibrio metschnikovii
<400> 5
   Ala Gln Gln Thr Pro Tyr Gly Ile Arg Met Val Gln Ala Asp Gln Leu
   1               5                   10                  15
   Ser Asp Val Tyr Ala Ala Asn Arg Lys Val Cys Val Ile Asp Ser Gly
               20                  25                  30
   Tyr Leu Arg Asn His Val Asp Leu Pro Ser Ala Gly Val Thr Gly Ser
           35                  40                  45
   Thr Phe Ser Gly His Gly Ser Trp Phe Thr Asp Gly Asn Gly His Gly
       50                  55                  60
   Thr His Val Ala Gly Thr Ile Val Ala Leu Asp Asn Asn Val Gly Val
   65                  70                  75                  80
   Val Gly Val Leu Pro Ser Gly Leu Val Gly Leu His Asn Val Lys Ile
                   85                  90                  95
   Phe Thr Asp Ser Gly Val Trp Thr Arg Ala Ser Asp Leu Ile Gln Ala
               100                 105                 110
   Ile Gln Ser Cys Gln Ser Ala Gly Ser His Val Val Asn Met Ser Leu
           115                 120                 125
   Gly Gly Ser Gln Gly Ser Val Thr Glu Gln Asn Ala Met Arg Asn Phe
       130                 135                 140
   Tyr Gln Gln Gly Met Leu Leu Val Ala Ala Ala Gly Asn Ser Gly Asn
   145                 150                 155                 160
   Ser Gly Phe Ser Tyr Pro Ala Ser Tyr Asp Ala Val Val Ser Val Ala
                   165                 170                 175
   Ala Val Asn Ser Ser Gly Asn Val Ala Asn Phe Ser Gln Phe Asn Ser
               180                 185                 190
   Gln Val Glu Leu Ser Ala Pro Gly Val Asn Val Leu Ser Thr Gly Asn
           195                 200                 205
   Asn Gly Gly Tyr Leu Ser Tyr Ser Gly Thr Ser Met Ala Ser Pro His
       210                 215                 220
   Val Ala Gly Val Ala Ala Leu Val Trp Ser His Phe Pro Gln Cys Arg
   225                 230                 235                 240
   Pro Glu Arg Ile Arg Gln Ser Leu Ser Gln Thr Ala Leu Asp Arg Gly
                   245                 250                 255
   Ala Ala Gly Arg Asp Asn Phe Tyr Gly Trp Gly Ile Val Gln Ala Arg
               260                 265                 270
   Ala Ala Tyr Asn Trp Leu Ser Arg Asn Gly Cys
           275                 280
<210> 6
   <211> 422
   <212> PRT
   <213> Vibrio metschnikovii
<220>
   <221> mat_peptide
   <222> (140)..(422)
<400> 6
   Met Leu Lys Lys Asn  Val Asn Arg Thr Val  Leu Ala Gly Leu Leu
                   -135                 -130                 -125
   Leu Pro Thr Ser Ile  Ser Leu Ala Val Ala  Ser Gln Leu Lys Asp
                   -120                 -115                 -110
   Gln Glu Val Pro Ser  Phe Thr Pro Ser Val  Ala Val Glu Asn His Gln
                   -105                 -100                 -95
   Thr Glu Gln Arg Tyr Phe Val Thr Tyr Val Pro Gly Ala Thr Ser Gly
               -90                 -85                 -80
   Pro Met Arg Met Ser Gln Asn Gly Leu Thr Glu Thr Asp Phe Ser Leu
           -75                 -70                 -65
   Gln Lys Ala Ala Asp Ile Leu Ser Thr Glu Gln Val Thr Ile Ile Asn
             -60                 -55                 -50
   His Leu Glu Ser Leu His Thr Ser Val Val Glu Met Thr Pro Ser Gln
   -45                 -40                 -35                 -30
   Ala Lys Gln Leu Leu Asp Asn Pro Asp Val Ala Met Ile Glu Val Asp
                   -25                 -20                 -15
   Pro Ile Arg Tyr Leu Phe Asp Ala Glu Ile Glu Pro Tyr Ala Gln Gln
               -10                 -5                  -1  1
   Thr Pro Tyr Gly Ile Arg Met Val Gln Ala Asp Gln Leu Ser Asp Val
       5                   10                  15
   Tyr Ala Ala Asn Arg Lys Val Cys Val Ile Asp Ser Gly Tyr Leu Arg
   20                  25                  30                  35
   Asn His Val Asp Leu Pro Ser Ala Gly Val Thr Gly Ser Thr Phe Ser
                   40                  45                  50
   Gly His Gly Ser Trp Phe Thr Asp Gly Asn Gly His Gly Thr His Val
               55                  60                  65
   Ala Gly Thr Ile Val Ala Leu Asp Asn Asn Val Gly Val Val Gly Val
           70                  75                  80
   Leu Pro Ser Gly Leu Val Gly Leu His Asn Val Lys Ile Phe Thr Asp
       85                  90                  95
   Ser Gly Val Trp Thr Arg Ala Ser Asp Leu Ile Gln Ala Ile Gln Ser
   100                 105                 110                 115
   Cys Gln Ser Ala Gly Ser His Val Val Asn Met Ser Leu Gly Gly Ser
                   120                 125                 130
   Gln Gly Ser Val Thr Glu Gln Asn Ala Met Arg Asn Phe Tyr Gln Gln
               135                 140                 145
   Gly Met Leu Leu Val Ala Ala Ala Gly Asn Ser Gly Asn Ser Gly Phe
           150                 155                 160
   Ser Tyr Pro Ala Ser Tyr Asp Ala Val Val Ser Val Ala Ala Val Asn
       165                 170                 175
   Ser Ser Gly Asn Val Ala Asn Phe Ser Gln Phe Asn Ser Gln Val Glu
   180                 185                 190                 195
   Leu Ser Ala Pro Gly Val Asn Val Leu Ser Thr Gly Asn Asn Gly Gly
                   200                 205                 210
   Tyr Leu Ser Tyr Ser Gly Thr Ser Met Ala Ser Pro His Val Ala Gly
               215                 220                 225
   Val Ala Ala Leu Val Trp Ser His Phe Pro Gln Cys Arg Pro Glu Arg
           230                 235                 240
   Ile Arg Gln Ser Leu Ser Gln Thr Ala Leu Asp Arg Gly Ala Ala Gly
       245                 250                 255
   Arg Asp Asn Phe Tyr Gly Trp Gly Ile Val Gln Ala Arg Ala Ala Tyr
   260                 265                 270                 275
   Asn Trp Leu Ser Arg Asn Gly Cys
                   280
<210> 7
   <211> 36
   <212> DNA
   <213> Artificial
<220>
   <223> forward PCR primer
<400> 7
   aaagaagact aagtaatgtt gaagaaaaat gtcaac 36
<210> 8
   <211> 37
   <212> DNA
   <213> Artificial
<220>
   <223> reverse PCR primer
<400> 8
   gaagacgtgt tattaacagc cattgcgaga taaccag 37

## Beschreibung

Die Erfindung betrifft Wasch- und Reinigungsmittel, enthaltend Proteasen, die für den Einsatz in Wasch- und Reinigungsmitteln geeignet sind, entsprechende Wasch- und Reinigungsverfahren sowie Verwendungen der Proteasen, insbesondere zu Wasch- und Reinigungszwecken.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Es ist ferner bekannt, dass einige für den Einsatz in Wasch- oder Reinigungsmitteln etablierte Proteasen auch für kosmetische Zwecke oder für die organisch-chemische Synthese geeignet sind.

In Wasch- und Reinigungsmitteln werden oftmals Proteasen vom Subtilisin-Typ (Subtilasen, Subtilopeptidasen, EC 3.4.21.62) und hierunter insbesondere Subtilisine eingesetzt. Sie wirken als unspezifische Endopeptidasen und hydrolysieren beliebige Säureamidbindungen, die im Inneren von Peptiden oder Proteinen liegen. Ihr pH-Optimum liegt meist im alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegeben von R. Bott und C. Betzel, New York, 1996. Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus Bacillus lentus, insbesondere aus Bacillus lentus DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7.

Viele Proteasen und hierunter insbesondere solche, die in Wasch- oder Reinigungsmitteln Verwendung finden, werden natürlicherweise von Mikroorganismen gebildet. Beispielsweise werden Subtilisine häufig von Bacillus-Spezies synthetisiert und sezerniert.

In Wasch- oder Reinigungsmitteln werden auch Proteasen eingesetzt, die aus anderen Mikroorganismen stammen, beispielsweise aus extremophilen Mikroorganismen. Diesbezüglich offenbart die koreanische Patentanmeldung KR 1999-12588 die Protease VapK aus Vibrio metschnikovii und deren Verwendung in Wasch- und Reinigungsmitteln. In der Veröffentlichung von Jellouli et al. (J. Ind. Microbiol. Biotechnol. (2009) 36:939-948) ist die Protease AprJ1 aus dem Bakterienstamm Vibrio metschnikovii J1 offenbart und deren mögliche Verwendung in Waschmitteln vorgeschlagen. Proteasen aus Vibrio metschnikovii sind ferner offenbart in den Veröffentlichungen von Kwon et al. (Kor. Jour. Microbiol. (1992), 501-506; Biotechnology Letters (1994) 16(4), 413-418; Gene (1995) 152, 59-63) und So-Sun et al. (J. Microbiol. Biotechnol. (2000), 10(3), 349-354).

Unterschiedliche Anwendungsbedingungen und -gebiete erfordern Proteasen mit unterschiedlichen Eigenschaften, was beispielsweise die Reaktionsbedingungen, die Stabilität oder auch die Substratspezifität angeht. Die Einsatzmöglichkeiten einer Protease, beispielsweise in einem Wasch- oder Reinigungsmittel, hängen von ihren enzymatischen Eigenschaften ab, beispielsweise von ihrem Temperatur- und pH-Profil, von ihrer katalytischen Aktivität in einer Waschflotte oder von ihrer Stabilität, insbesondere gegenüber hohen oder niedrigen Temperaturen, oxidierenden Agentien oder Tensiden. Ferner sind Faltungseffekte oder erwünschte Synergien mit anderen Inhaltsstoffen von Bedeutung. Es besteht folglich weiterhin ein hoher Bedarf an neuen Proteasen mit vorteilhaften enzymatischen Eigenschaften, insbesondere für den Einsatz in Wasch- und Reinigungsmitteln.

Der vorliegenden Erfindung liegt die Aufgabe zugrunde, weitere, bislang noch nicht bekannte Proteasen bereitzustellen, die für den Einsatz in Wasch- und Reinigungsmitteln geeignet sind.

Gegenstand der Erfindung ist eine Protease umfassend eine Aminosäuresequenz, die ausgewählt ist aus der Gruppe bestehend aus
a) Aminosäuresequenz, die zu der in SEQ ID NO. 3 angegebenen Aminosäuresequenz identisch ist.
b) Aminosäuresequenz, die zu der in SEQ ID NO. 2 angegebenen Aminosäuresequenz identisch ist.

Mit einer solchen Protease sind als Erfindungsgegenstände Wasch- und Reinigungsmittel, Wasch- und Reinigungsverfahren und über die Wasch-und Reinigungsmittel definierte Verwendungsmöglichkeiten verbunden.

Die der vorliegenden Erfindung zugrunde liegende, natürlicherweise gebildete Protease ist aus dem Kulturüberstand eines Bakterienstammes erhältlich, der von der DSMZ (DSMZ - Deutsche Sammlung von Mikroorganismen und Zellkulturen GmbH, Inhoffenstraße 7 B, 38124 Braunschweig, Deutschland) als ein Vibrio metschnikovii Stamm identifiziert ist (DSM ID 10-62). Der Stamm bzw. die ihn enthaltende Bodenprobe stammt aus der Volksrepublik China. Gemäß Budapester Vertrag wurde ein Plasmid, das die Nukleinsäuresequenz der erfindungsgemäßen Protease enthält, bei der DSMZ mit der Hinterlegungsnummer DSM 23708 am 18.06.2010 hinterlegt.

Eine erfindungsgemäße Protease weist eine proteolytische Aktivität auf, das heißt, sie ist zur Hydrolyse von Peptidbindungen eines Polypeptids bzw. Proteins befähigt, insbesondere in einem Wasch- oder Reinigungsmittel bzw. in einer durch das Mittel gebildeten Waschflotte.

Überraschenderweise wurde gefunden, dass erfindungsgemäße Proteasen einen so guten Beitrag zur Reinigungsleistung eines Wasch- oder Reinigungsmittels, welches die Protease enthält, leisten, der dem Beitrag von einem für diesen Zweck etablierten proteolytischen Enzym zur Reinigungsleistung des Mittels nahe kommt und ihn an unterschiedlichen Anschmutzungen sogar übertrifft. Dies gilt selbst für Ausführungsformen erfindungsgemäßer Proteasen, die Wildtyp-Formen sind. Erfindungsgemäße Proteasen ermöglichen folglich eine verbesserte Entfernung von mindestens einer, bevorzugt von mehreren protease-sensitiven Anschmutzungen auf Textilien und/oder harten Oberflächen, beispielsweise Geschirr. Besonders vorteilhafte Reinigungsleistungen zeigen bevorzugte Ausführungsformen erfindungsgemäßer Proteasen an Gras- und/oder Kakao-haltigen Anschmutzungen, beispielsweise den Anschmutzungen Gras auf Baumwolle: Produkt Nr. 164 der Firma Eidgenössische Material- und Prüfanstalt (EMPA) Testmaterialien AG (St. Gallen, Schweiz) und/oder Kakao auf Baumwolle: Produkt Nr. 112 der Firma Eidgenössische Material- und Prüfanstalt (EMPA) Testmaterialien AG (St. Gallen, Schweiz). Folglich werden durch bevorzugte Ausführungsformen der vorliegenden Erfindung schmutzspezifische Proteasen bereitgestellt, deren Reinigungsleistung gezielt im Hinblick auf eine Anschmutzung oder auf mehrere Anschmutzungen ähnlichen Typs vorteilhaft ist.

Bevorzugte Ausführungsformen erfindungsgemäßer Proteasen erzielen solche vorteilhaften Reinigungsleistungen auch schon bei niedrigen Temperaturen zwischen 10°C und 40°C, zwischen 10°C und 30°C und zwischen 10°C und 25°C, beispielsweise bei 20°C.

Ferner verfügen bevorzugte Ausführungsformen erfindungsgemäßer Proteasen über eine besondere Stabilität gegenüber Tensiden und/oder Bleichmitteln und/oder gegenüber Temperatureinflüssen, insbesondere gegenüber hohen oder niedrigen Temperaturen und/oder gegenüber sauren oder alkalischen Bedingungen und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse.

Unter Reinigungsleistung wird die Aufhellungsleistung eines Wasch- oder Reinigungsmittels an Anschmutzungen, insbesondere an Protease-sensitiven Anschmutzungen und hierunter insbesondere an Protease-sensitiven Wäscheanschmutzungen, verstanden. Die Reinigungsleistung wird bevorzugt ermittelt wie weiter unten angegeben.

Zahlreiche Proteasen werden als sogenannte Präproteine, also zusammen mit einem Propeptid und/oder einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins, wobei Signal- und Propeptid sich auch überlappen oder im Extremfall sogar zusammenfallen können. Nach der Abspaltung von Signal- und Propeptid übt die dann reife (mature) Protease ihre katalytische Aktivität ohne die ursprünglich vorhandenen N-terminalen Aminosäuren aus.

Eine Nukleinsäuresequenz ist unter SEQ ID NO. 1 angegeben. Diese Nukleinsäure codiert für eine Protease, welche eine für Proteasen typische Einteilung in Signal- und/oder Propeptid und reife (mature) Protease aufweist. Das Gesamtlängenprotein ist unter SEQ ID NO. 2 und die reife (mature) Protease unter SEQ ID NO. 3 angegeben. Erfindungsgemäß bevorzugt sind die reifen Proteasen. Diese weisen ein Molekulargewicht zwischen 27 und 33 kD (Kilodalton) auf, insbesondere zwischen 29 und 31 kD, ermittelt durch SDS-Polyacrylamidgelelektrophorese, während das Präprotein ein Molekulargewicht zwischen 42 und 47 kD, insbesondere zwischen 44 und 45 kD, aufweist.

Für die Protease gemäß SEQ ID NO. 3 bzw. SEQ ID NO. 2 wurde eine Sequenzanalyse sowie ein Sequenzvergleich mit bekannten Proteinsequenzen aus den allgemein zugänglichen Datenbanken UniProtKB und/oder Swiss-Prot (vgl. UniProtKB, EMBL Outstation - European Bioinformatics Institute, Wellcome Trust Genome Campus, Hinxton, Cambridge CB10 1SD, United Kingdom; http://www.uniprot.org) und/oder GenBank (National Center for Biotechnology Information NCBI, National Institutes of Health, Bethesda, MD, USA) durchgeführt, um die Proteine mit der größten Ähnlichkeit zu ermitteln. Für die Nukleinsäuresequenz gemäß SEQ ID NO. 1 wurde entsprechend ein Sequenzvergleich mit bekannten Nukleinsäuresequenzen durchgeführt.

Dieser Sequenzvergleich erfolgte basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. In der vorliegenden Patentanmeldung wurden alle Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Struktur, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

Durch einen solchen Vergleich mit bekannten Enzymen, die beispielsweise in allgemein zugänglichen Datenbanken hinterlegt sind, lässt sich für eine Aminosäuresequenz ferner deren enzymatische Aktivität folgern.

Für die reife (mature) Protease gemäß SEQ ID NO. 3 wurde auf Aminosäureebene über die Gesamtlänge von SEQ ID NO. 3 als nächstähnliches Protein identifiziert: alkalische Protease aus Vibrio metschnikovii Stamm J1 (VapK; Gen vapK) mit 97,2% Identität bzw. Abweichungen in acht Aminosäurepositionen (Datenbank Zugangsnummer (Accession) EU443196). Diese Sequenz ist angegeben als SEQ ID NO. 5. Die Angaben sind jeweils bezogen sind auf die reife Protease.

Für die gesamte Protease gemäß SEQ ID NO. 2 wurde auf Aminosäureebene über die Gesamtlänge von SEQ ID NO. 2 ebenfalls die alkalische Protease aus Vibrio metschnikovii Stamm J1 (VapK; Gen vapK) mit 96,9% Identität bzw. Abweichungen in 13 Aminosäurepositionen als nächstähnliches Protein identifiziert. Diese Sequenz ist angegeben als SEQ ID NO. 6. Die Angaben sind jeweils bezogen sind auf die gesamte Protease.

Zu im Stand der Technik etablierten Proteasen für Wasch- und Reinigungsmittel ergeben sich folgende Identitäten: 42% zur alkalischen Protease aus Bacillus lentus (angegeben als SEQ ID NO. 4, vgl. auch WO 97/21760 A1) und 40% zur Protease aus Bacillus amyloliquefaciens BPN', jeweils bezogen auf das reife Enzym.

Die alkalische Protease aus Vibrio metschnikovii Stamm J1 (VapK; Gen vapK; Datenbankeintrag EU443196) ist in SEQ ID NO. 5 dargestellt.

Die alkalische Protease aus Vibrio metschnikovii Stamm J1 (VapK; Gen vapK; Datenbankeintrag EU443196) ist in SEQ ID NO. 6 dargestellt.

In einer weiteren Ausführungsform der Erfindung ist die Protease dadurch gekennzeichnet, dass ihre Reinigungsleistung mindestens derjenigen einer Protease entspricht, die eine Aminosäuresequenz gemäß SEQ ID NO. 3 aufweist, und/oder mindestens derjenigen einer Protease entspricht, die eine Aminosäuresequenz gemäß SEQ ID NO. 5 aufweist, wobei die Reinigungsleistung in einem Waschsystem bestimmt wird, das aus einem Waschmittel und der Protease gebildet wird, wobei die zu vergleichenden Proteasen aktivitätsgleich eingesetzt sind und die Reinigungsleistung gegenüber einer oder mehrerer der Anschmutzungen Blut-Milch/Tusche auf Baumwolle, Erdnussöl-Pigment/Milch auf Polyester/Baumwolle, Gras auf Baumwolle und Kakao auf Baumwolle, insbesondere gegenüber einer oder mehrerer der Anschmutzungen
(1) Blut-Milch/Tusche auf Baumwolle: Produkt Nr. C-05 erhältlich von CFT (Center For Testmaterials) B.V. Vlaardingen, Niederlande,
(2) Erdnussöl-Pigment/Milch auf Polyester/Baumwolle: Produkt Nr. PC-10 erhältlich von CFT (Center For Testmaterials) B.V. Vlaardingen, Niederlande,
(3) Gras auf Baumwolle: Produkt Nr. 164 erhältlich von der Firma Eidgenössische Material- und Prüfanstalt (EMPA) Testmaterialien AG, St. Gallen, Schweiz,
(4) Kakao auf Baumwolle: Produkt Nr. 112 erhältlich von der Firma Eidgenössische Material- und Prüfanstalt (EMPA) Testmaterialien AG, St. Gallen, Schweiz,
bestimmt wird durch Messung des Weißheitsgrades der gewaschenen Textilien, der Waschvorgang für mindestens 30 Minuten, optional 60 Minuten, bei einer Temperatur von 20°C erfolgt und das Wasser eine Wasserhärte zwischen 15,5 und 16,5° (deutsche Härte) aufweist.

Ein bevorzugtes flüssiges Waschmittel für ein solches Waschsystem ist wie folgt zusammengesetzt (alle Angaben in Gewichts-Prozent): 0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1% Borsäure, 1-2% Natriumcitrat (Dihydrat), 2-4% Soda, 14-16% Kokosnuss-Fettsäuren, 0,5% HEDP (1-Hydroxyethan-(1,1-di-phosphonsäure)), 0-0,4% PVP (Polyvinylpyrrolidon), 0-0,05% optischer Aufheller, 0-0,001 % Farbstoff, Rest demineralisiertes Wasser. Die Dosierung des flüssigen Waschmittels beträgt zwischen 2,0 und 9,0, vorzugsweise zwischen 3,0 und 8,2, zwischen 4,0 und 7,5 und besonders bevorzugt 4,7 Gramm pro Liter Waschflotte. Gewaschen wird in einem pH-Wertebereich zwischen pH 8 und pH 10,5, bevorzugt zwischen pH 8 und pH 9.

Ein bevorzugtes pulverförmiges Waschmittel für ein solches Waschsystem ist wie folgt zusammengesetzt (alle Angaben in Gewichts-Prozent): 10% lineares Alkylbenzolsulfonat (Natrium-Salz), 1,5% C12-C18-Fettalkoholsulfat (Natrium-Salz), 2,0% C12-C18-Fettalkohol mit 7 EO, 20% Natriumcarbonat, 6,5% Natriumhydrogencarbonat, 4,0% amorphes Natriumdisilikat, 17% Natriumcarbonat-peroxohydrat, 4,0% TAED, 3,0% Polyacrylat, 1,0% Carboxymethylcellulose, 1,0% Phosphonat, 25% Natriumsulfat, Rest: optional Schauminhibitoren, optischer Aufheller, Duftstoffe und ggfs. Wasser ad 100%. Die Dosierung des pulverförmigen Waschmittels beträgt zwischen 4,5 und 7,0 Gramm und vorzugsweise zwischen 5,5 und 7,0 Gramm pro Liter Waschflotte. Gewaschen wird in einem pH-Wertebereich zwischen pH 9 und pH 11.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Im Rahmen der Erfindung erfolgt die Bestimmung die Reinigungsleistung bevorzugt bei 20°C gegenüber der Anschmutzung (3) und/oder (4) unter Verwendung eines pulverförmigen Waschmittels wie vorstehend angegeben und wie in Beispiel 2 ausgeführt.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann. Verfahren zur Bestimmung der Proteaseaktivität sind dem Fachmann auf dem Gebiet der Enzymtechnologie geläufig und werden von ihm routinemäßig angewendet. Beispielsweise sind solche Verfahren offenbart in Tenside, Band 7 (1970), S. 125-132.

Alternativ kann die Proteaseaktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s.

Die Proteaseaktivität wird üblicherweise in Protease-Einheiten (PE) angegeben. Geeignete Proteaseaktivitäten betragen beispielsweise 2,25, 5 oder 10 PE pro ml Waschflotte. Die Proteaseaktivität ist jedoch nicht gleich Null.

In einer weiteren Ausführungsform der Erfindung ist eine erfindungsgemäße Protease natürlicherweise in einem Mikroorganismus vorhanden. Diese Ausführungsform ist deshalb besonders vorteilhaft, weil dann der zugehörige Organismus selbst in Kultur genommen werden kann.

Vorteilhafterweise lassen sich dann aus dessen Zellextrakten oder Kulturüberständen erfindungsgemäße Proteasen isolieren und herstellen. Die Protease ist vorhanden in Vibrio metschnikovii und insbesondere in Vibrio metschnikovii DSM ID 10-62.
Ferner wurde eine für eine bevorzugte erfindungsgemäße Protease codierende Nukleinsäure im Rahmen der vorliegenden Erfindung rekombinant in eine Wirtszelle eingebracht und wie vorstehend beschrieben unter der Nummer DSM 23708 am 18.06.2010 bei der DSMZ hinterlegt. In einer weiteren bevorzugten Ausführungsform der Erfindung ist die Protease somit dadurch gekennzeichnet, dass sie von einer Nukleinsäure codiert wird, die in einer Wirtszelle mit der Hinterlegungsnummer DSM 23708 vorhanden ist.
Proteine können über die Reaktion mit einem Antiserum oder einem bestimmten Antikörper zu Gruppen immunologisch verwandter Proteine zusammengefasst werden. Die Angehörigen einer solchen Gruppe zeichnen sich dadurch aus, dass sie dieselbe, von einem Antikörper erkannte antigene Determinante aufweisen. Sie sind daher einander strukturell so ähnlich, dass sie von einem Antiserum oder bestimmten Antikörpern erkannt werden. Einen weiteren Erfindungsgegenstand bilden daher Proteasen, die dadurch gekennzeichnet sind, dass sie mindestens eine und zunehmend bevorzugt zwei, drei oder vier übereinstimmende antigene Determinanten mit einer erfindungsgemäßen Protease wie vorstehend beschrieben aufweisen. Solche Proteasen sind auf Grund ihrer immunologischen Übereinstimmungen den erfindungsgemäßen Proteasen strukturell so ähnlich, dass auch von einer gleichartigen Funktion auszugehen ist.

Zusätzlich zu den vorstehend erläuterten Aminosäureveränderungen können Proteasen weitere Aminosäureveränderungen, insbesondere AminosäureSubstitutionen, -Insertionenen oder -Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden.
Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease, zum Beispiel hinsichtlich ihrer Stabilität gegenüber Tensiden und/oder Bleichmitteln und/oder anderen Komponenten, kann daher im Rahmen der Erfindung zusätzlich weiterentwickelt sein.
Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt. Beispielsweise beschreibt A95G die Substitution von Alanin an Position 95 durch Glycin, A95AG die Insertion von Glycin nach der Aminosäure Alanin an Position 95 und A95* die Deletion von Alanin an Position 95. Diese Nomenklatur ist dem Fachmann auf dem Gebiet der Enzymtechnologie bekannt. Im Sinne der vorliegenden Erfindung können ferner Enzyme, Proteine, Fragmente und Derivate, sofern sie nicht explizit als solche angesprochen zu werden brauchen, unter dem Oberbegriff Proteine bzw. Polypeptide zusammengefasst werden. Es werden Protease beschrieben, die dadurch gekennzeichnet ist, dass sie aus einer Protease wie vorstehend beschrieben als Ausgangsmolekül erhaltbar ist durch ein- oder mehrfache konservative Aminosäuresubstitution. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T. Vorzugsweise ist eine derart erhaltene Protease noch mindestens zu 60% und zunehmend bevorzugt zu 65%, 70%, 75%, 80%, 85%, 87,5%, 90%, 92,5%, 95%, 96% identisch zu SEQ ID NO. 3 und/oder weist an mindestens einer und zunehmend bevorzugt an 2, 3, 4, 5, 6, 7 und besonders bevorzugt an 8 der Positionen 38, 98, 162, 184, 190, 202, 249 und 263 in der Zählung gemäß SEQ ID NO. 3 diejenige Aminosäure auf, die in SEQ ID NO. 3 an dieser Position vorhanden ist.

Alternativ oder ergänzend ist die Protease dadurch gekennzeichnet, dass sie aus einer erfindungsgemäßen Protease als Ausgangsmolekül erhaltbar ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270, 275 oder 280 zusammenhängenden Aminosäuren mit dem Ausgangsmolekül übereinstimmt.

So ist es beispielsweise möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms. Auch Substitutionen können vorteilhafte Wirkungen zeigen. Sowohl einzelne wie auch mehrere zusammenhängende Aminosäuren können gegen andere Aminosäuren ausgetauscht werden. Es werden Proteasen beschrieben, die zusätzlich stabilisiert ist, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt. So können Proteasen beispielsweise auch dadurch stabilisiert werden, dass einer oder mehrere Tyrosin-Reste gegen andere Aminosäuren ausgetauscht werden.
Weitere Möglichkeiten der Stabilisierung sind beispielsweise:
- Veränderung der Bindung von Metallionen, insbesondere der Calcium-Bindungsstellen, beispielsweise durch Austauschen von einer oder mehreren der an der Calcium-Bindung beteiligten Aminosäure gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf bzw. an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.
Bevorzugte Ausführungsformen sind solche, bei denen das Enzym auf mehrere Arten stabilisiert wird, da mehrere stabilisierende Mutationen additiv oder synergistisch wirken. Es werden Proteasen beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert.
Unter Derivaten werden im Sinne der vorliegenden Anmeldung demnach solche Proteine verstanden, deren reine Aminosäurekette chemisch modifiziert worden ist. Solche Derivatisierungen können beispielsweise in vivo durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der das Protein produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen-eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Proteasen mit Protease-Inhibitoren ist vorteilhaft.
Betreffend alle vorstehend beschriebenen Proteasen bzw. Proteasevarianten und/oder Derivate sind diejenigen besonders bevorzugt, deren Aktivität mindestens derjenigen der Protease gemäß SEQ ID NO. 3 entspricht, und/oder deren Reinigungsleistung mindestens derjenigen der Protease gemäß SEQ ID NO. 3 entspricht, wobei die Reinigungsleistung in einem Waschsystem bestimmt wird wie vorstehend beschrieben. Die Bestimmung der Proteaseaktivität ist eine fachübliche Maßnahme und erfolgt diesbezüglich wie vorstehend beschrieben.

Alle vorstehend ausgeführten Sachverhalte können zu Verfahren kombiniert werden, um erfindungsgemäße Proteasen herzustellen.

Da erfindungsgemäße Proteasen vorteilhafte Reinigungsleistungen insbesondere an Gras- und/oder Kakao-haltigen Anschmutzungen aufweisen, sind die Wasch- und Reinigungsmittel der vorliegenden Erfindung insbesondere zur Entfernung derartigen Anschmutzungen geeignet und vorteilhaft.

Zu diesem Erfindungsgegenstand zählen alle denkbaren Wasch- bzw. Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als insbesondere pulverförmige Feststoffe, in nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Protease mit einem oder mehreren weiteren Inhaltsstoff(en) des Mittels ist vorteilhaft, da ein solches Mittel eine verbesserte Reinigungsleistung durch sich ergebende Synergismen aufweist. Insbesondere durch die Kombination einer erfindungsgemäßen Protease mit einem Tensid und/oder einem Builder (Gerüststoff) und/oder einer Persauerstoffverbindung und/oder einem Bleichaktivator kann ein solcher Synergismus erreicht werden.

Vorteilhafte Inhaltsstoffe erfindungsgemäßer Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein erfindungsgemäßes Mittel enthält die Protease vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels. Ferner kann die in dem Mittel enthaltene Protease, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) als Einkomponentensystem vorliegt, oder
(d) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäße Wasch- oder Reinigungsmittel können ausschließlich eine Protease enthalten. Alternativ können sie auch weitere hydrolytische Enzyme oder andere Enzyme in einer für die Wirksamkeit des Mittels zweckmäßigen Konzentration enthalten. Einen weiteren Gegenstand der Erfindung stellen somit Mittel dar, die ferner eines oder mehrere weitere Enzyme umfassen, wobei prinzipiell alle im Stand der Technik für diese Zwecke etablierten Enzyme einsetzbar sind. Als weitere Enzyme bevorzugt einsetzbar sind alle Enzyme, die in dem erfindungsgemäßen Mittel eine katalytische Aktivität entfalten können, insbesondere eine insbesondere eine Protease, Amylase, Cellulase, Hemicellulase, Mannanase, Pektinase, Tannase, Xylanase, Xanthanase, β-Glucosidase, Carrageenase, Perhydrolase, Oxidase, Oxidoreduktase oder eine Lipase sowie Kombinationen hieraus. Diese Enzyme sind im Prinzip natürlichen Ursprungs; ausgehend von den natürlichen Molekülen stehen für den Einsatz in Wasch- und Reinigungsmitteln auch verbesserte Varianten zur Verfügung, die entsprechend bevorzugt eingesetzt werden.

Erfindungsgemäße Mittel enthalten Enzyme vorzugsweise in Gesamtmengen von 1 x 10⁻⁸ bis 5 Gewichts-Prozent bezogen auf aktives Protein. Bevorzugt sind die Enzyme von 0,001 bis 5 Gew.-%, weiter bevorzugt von 0,01 bis 5 Gew.-%, noch weiter bevorzugt von 0,05 bis 4 Gew.-% und besonders bevorzugt von 0,075 bis 3,5 Gew.-% in erfindungsgemäßen Mitteln enthalten, wobei jedes enthaltene Enzym in den genannten Mengenverhältnissen vorliegen kann. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden.

Besonders bevorzugt unterstützen die weiteren Enzyme die Wirkung des Mittels, beispielsweise die Reinigungsleistung eines Wasch- oder Reinigungsmittels, hinsichtlich bestimmter Anschmutzungen oder Flecken. Besonders bevorzugt zeigen die Enzyme synergistische Effekte hinsichtlich ihrer Wirkung gegenüber bestimmter Anschmutzungen oder Flecken, d.h. die in der Mittelzusammensetzung enthaltenen Enzyme unterstützen sich in ihrer Reinigungsleistung gegenseitig. Derartige Synergismen treten insbesondere zwischen einer erfindungsgemäßen Protease und einer Amylase und/oder einer Cellulase und/oder einer Lipase und/oder einer Pektinase auf. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Protease katalytisch aktiv ist, insbesondere derart, dass die Protease in einer Menge von 40 µg bis 4 g, vorzugsweise von 50 µg bis 3 g, besonders bevorzugt von 100 µg bis 2 g und ganz besonders bevorzugt von 200 µg bis 1 g eingesetzt wird.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung bzw. Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Protease bereichert werden und stellen dann Ausführungsformen der vorliegenden Erfindung dar. Alle Sachverhalte, Gegenstände und Ausführungsformen, die für erfindungsgemäße Proteasen bzw. sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da erfindungsgemäße Proteasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einziger Schritt eines solchen Verfahrens darin bestehen, dass gewünschtenfalls als einzige reinigungsaktive Komponente eine solche Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Ein weiterer Erfindungsgegenstand ist die Verwendung eines erfindungsgemäßen Mittels zur Reinigung von Textilien oder von harten Oberflächen, oder einer erfindungsgemäßen Protease zur Reinigung von Textilien oder von harten Oberflächen, insbesondere derart, dass die Protease in einer Menge von 40 µg bis 4 g, vorzugsweise von 50 µg bis 3 g, besonders bevorzugt von 100 µg bis 2 g und ganz besonders bevorzugt von 200 µg bis 1 g eingesetzt wird.

Alle Sachverhalte, Gegenstände und Ausführungsformen, die für erfindungsgemäße Proteasen bzw. sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

### Beispiel 1

### Isolierung und Identifizierung des proteolytisch aktiven Bakterienstamms

0,1g einer Probe aus dem Uferbereich des Dali Lake (Volksrepublik China) wurden in 1ml sterilem Kulturmedium (5g/l NaCl; 0,2g/l MgSO₄ x 7 H₂O; 1g/l K₂HPO₄; 0,2 g/l KCl; 5g/l Hefeextrakt; 5g/l Polypepton; 5g/l Glucose; 10g/l Na₂CO₃; pH 10) suspendiert, für 48h bei 30°C inkubiert und anschließend auf milchpulverhaltigen Medium-Agarplatten (Kulturmedium zuzüglich 1,5% Agar, 1% Magermilchpulver) ausplattiert und für 48h bei 30°C inkubiert. Anhand des Klärungshofes auf der Agarplatte wurde ein proteolytisch aktiver Stamm der Bakterienart Vibrio metschnikovii (DSM ID 10-62) identifiziert.

### Beispiel 2

### Klonierung der Protease

Das proteolytisch aktive Bakterium wurde in Kulturmedium wie in Beispiel 1 beschrieben für 48h bei 30°C kultiviert. Die Gesamt-DNA dieses Bakteriums wurde nach Standardmethoden präpariert, die für die Protease codierende Nukleinsäuresequenz mit Hilfe der Polymerase-Kettenreaktion unter Verwendung der in SEQ ID NO. 7 und SEQ ID NO. 8 angegebenen Oligonukleotide als Primer amplifiziert und als Insert in den E. coli Vektor pUC19 kloniert. Dieses Insert enthält einen offenen Leserahmen von ca. 1,3 kb, dessen DNA-Sequenz für eine alkalische Protease codiert. Dieser Vektor wurde gemäß dem Budapester Vertrag am 18.06.2010 bei der DSMZ unter der Nummer DSM 23708 hinterlegt.

### Beispiel 3

### Ermittlung der Reinigungsleistung bei Einsatz in einem pulverförmigen Waschmittel

Für dieses Beispiel wurden standardisiert verschmutzte Textilien eingesetzt. Es wurden folgende Anschmutzung und Textilien verwendet:
(1) Blut-Milch/Tusche auf Baumwolle: Produkt Nr. C-05 erhältlich von CFT (Center For Testmaterials) B.V. Vlaardingen, Niederlande
(2) Erdnussöl-Pigment/Milch auf Polyester/Baumwolle: Produkt Nr. PC-10 erhältlich von CFT (Center For Testmaterials) B.V. Vlaardingen, Niederlande
(3) Gras auf Baumwolle: Produkt Nr. 164 erhältlich von der Firma Eidgenössische Material- und Prüfanstalt (EMPA) Testmaterialien AG, St. Gallen, Schweiz
(4) Kakao auf Baumwolle: Produkt Nr. 112 erhältlich von der Firma Eidgenössische Material- und Prüfanstalt (EMPA) Testmaterialien AG, St. Gallen, Schweiz.

Mit diesem Testmaterial wurden verschiedene Waschmittelrezepturen auf ihre Reinigungsleistung hin untersucht. Dafür wurden die Ansätze für 60 Minuten bei einer Temperatur von 20°C oder 40°C gewaschen. Die Dosierung lag bei 5,9 g des Waschmittels pro Liter Waschflotte. Es wurde mit Stadtwasser mit einer Wasserhärte von 16° deutscher Härte gewaschen.

Als Waschmittel wurde ein pulverförmiges Waschmittel verwendet, das folgende Zusammensetzung aufwies (alle Angaben in Gewichts-Prozent): 10% lineares Alkylbenzolsulfonat (Natrium-Salz), 1,5% C12-C18-Fettalkoholsulfat (Natrium-Salz), 2,0% C12-C18-Fettalkohol mit 7 EO, 20% Natriumcarbonat, 6,5% Natriumhydrogencarbonat, 4,0% amorphes Natriumdisilikat, 17% Natriumcarbonat-peroxohydrat, 4,0% TAED, 3,0% Polyacrylat, 1,0% Carboxymethylcellulose, 1,0% Phosphonat, 25% Natriumsulfat, Rest: Schauminhibitoren, optischer Aufheller, Duftstoffe. Das Waschmittel wurde für die verschiedenen Versuchsreihen aktivitätsgleich (5 PE/ml Endkonzentration) mit einer erfindungsgemäßen Protease umfassend eine Aminosäuresequenz gemäß SEQ ID NO. 3 und einer leistungsverbesserten Variante der alkalischen Protease aus Bacillus lentus gemäß WO 92/21760 A1, einer für Wasch- und Reinigungsmittel etablierten Protease, als Kontrolle (Ansatz 2) versetzt.

Nach dem Waschen wurde der Weißheitsgrad der gewaschenen Textilien gemessen. Die Messung erfolgte an einem Spektrometer Minolta CM508d (Lichtart D65, 10°). Das Gerät wurde zuvor mit einem mitgelieferten Weißstandard kalibriert. Die erhaltenen Ergebnisse sind die Differenzremissionen zwischen einem Waschvorgang mit einem Waschmittel enthaltend eine Protease und einem parallel durchgeführten Kontrollwaschgang mit einem Waschmittel ohne Protease. Die Ergebnisse sind in der nachfolgenden Tabelle 1 zusammengestellt und erlauben einen unmittelbaren Rückschluss auf den Beitrag der jeweils enthaltenen Protease zur Reinigungsleistung des verwendeten Mittels.

**Tabelle 1: Waschergebnisse mit einem pulverförmigen Waschmittel bei 20°C und 40°C**

| | 20°C | | 40°C | |
|---|---|---|---|---|
| Anschmutzung | Ansatz 1 | Ansatz 2 | Ansatz 1 | Ansatz 2 |
| (1) | 8,3 | 6,6 | 10,8 | 10,0 |
| (2) | 10,8 | 11,0 | 11,1 | 10,7 |
| (3) | 2,2 | 0,8 | 4,8 | 4,2 |
| (4) | 5,1 | 4,0 | 4,8 | 2,8 |

Es wird deutlich, dass eine erfindungsgemäße Protease bereits in ihrer Wildtyp-Form eine bessere Reinigungsleistung zeigt im Vergleich zu einer für Waschmittel etablierten leistungsverbesserten Proteasevariante gemäß Ansatz 2, die kein Wildtyp-Molekül ist.

### Beschreibung der Figur:

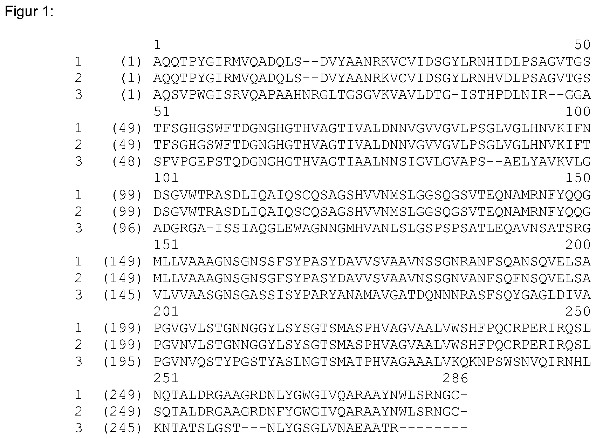

- Figur 1:: Sequenzvergleich (Alignment) der erfindungsgemäßen reifen Protease (SEQ ID NO. 3) mit Proteasen aus dem Stand der Technik, erstellt mit dem Programm Vector NTI® Suite 10.3 (Invitrogen Corporation, 1600 Faraday Avenue, Carlsbad, Kalifornien, USA) unter Standardparametern.

### Darin bedeuten:

1: Erfindungsgemäße Protease gemäß SEQ ID NO. 3 (reifes Enzym)
2: Alkalische Protease aus Vibrio metschnikovii Stamm J1 (VapK) (reifes Enzym, vgl. SEQ ID NO. 5)
3: Protease aus Bacillus lentus DSM 5483 (reifes Enzym, vgl. auch WO 92/21760 A1 bzw. SEQ ID NO. 4)

## Patentansprüche

1. Wasch- oder Reinigungsmittel, **dadurch gekennzeichnet, dass** es mindestens eine Protease enthält, die Protease umfassend eine Aminosäuresequenz, die ausgewählt ist aus der Gruppe bestehend aus
a) Aminosäuresequenz, die zu der in SEQ ID NO. 3 angegebenen Aminosäuresequenz identisch ist
b) Aminosäuresequenz, die zu der in SEQ ID NO. 2 angegebenen Aminosäuresequenz identisch ist.

2. Wasch- oder Reinigungsmittel nach Anspruch 1, **dadurch gekennzeichnet, dass** die Protease natürlicherweise in einem Mikroorganismus vorhanden ist, vorzugsweise in einem Pilz, in einem gramnegativen oder in einem grampositiven Bakterium, hierunter bevorzugt in einem Bakterium der Gattung Vibrio, insbesondere in Vibrio metschnikovii und insbesondere in Vibrio metschnikovii DSM ID 10-62,
oder dass sie von einer Nukleinsäure codiert wird, die in einer Wirtszelle mit der Hinterlegungsnummer DSM 23708 vorhanden ist.

3. Wasch- oder Reinigungsmittel nach einem der Ansprüche 1 bis 2, **dadurch gekennzeichnet, dass** es die Protease in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Wasch- oder Reinigungsmittel enthält,
und/oder dass die Protease in dem Wasch- oder Reinigungsmittel mit einer bei Raumtemperatur oder bei Abwesenheit von Wasser für die Protease undurchlässigen Substanz umhüllt ist.

4. Wasch- oder Reinigungsmittel nach Anspruch 3, **dadurch gekennzeichnet, dass** es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) als Einkomponentensystem vorliegt, oder
(d) in mehrere Komponenten aufgeteilt ist.

5. Wasch- oder Reinigungsmittel nach einem der Ansprüche 1 bis 4, **dadurch gekennzeichnet, dass** es eines oder mehrere weitere Enzyme umfasst, insbesondere eine Protease, Amylase, Cellulase, Hemicellulase, Mannanase, Pektinase, Tannase, Xylanase, Xanthanase, β-Glucosidase, Carrageenase, Perhydrolase, Oxidase, Oxidoreduktase oder eine Lipase sowie Kombinationen hieraus.

6. Verfahren zur Reinigung von Textilien oder harten Oberflächen, **dadurch gekennzeichnet, dass** in mindestens einem Verfahrensschritt ein Wasch- oder Reinigungsmittel nach einem der Ansprüche 1 bis 5 angewendet wird, insbesondere derart, dass die Protease in einer Menge von 40 µg bis 4 g, vorzugsweise von 50 µg bis 3 g, besonders bevorzugt von 100 µg bis 2 g und ganz besonders bevorzugt von 200 µg bis 1 g eingesetzt wird.

7. Verwendung eines Wasch- oder Reinigungsmittel nach einem der Ansprüche 1 bis 5 zur Reinigung von Textilien oder von harten Oberflächen, insbesondere derart, dass die in dem Wasch- oder Reinigungsmittel enthaltene Protease in einer Menge von 40 µg bis 4 g, vorzugsweise von 50 µg bis 3 g, besonders bevorzugt von 100 µg bis 2 g und ganz besonders bevorzugt von 200 µg bis 1 g eingesetzt wird.

## Claims

1. A washing or cleaning agent, **characterized in that** it contains at least one protease, the protease comprising an amino acid sequence, which is selected from the group consisting of
a) amino acid sequence, which is identical to the amino acid sequence shown in SEQ ID No. 3
b) amino acid sequence, which is identical to the amino acid sequence shown in SEQ ID No. 2.

2. The washing or cleaning agent according to claim 1, **characterized in that** the protease is naturally present in a microoganism, preferably in a fungus, in a gram-negative or in a gram-positive bacterium, including preferably in a bacterium of the genus Vibrio, in particular in Vibrio metschnikovii and in particular in Vibrio metschnikovii DSM ID 10-62,
or **in that** said protease is coded by a nucleic acid which is present in a host cell having the accession number DSM 23708.

3. The washing or cleaning agent according to one of claims 1 to 2, **characterized in that** it contains the protease in an amount of from 2 µg to 20 mg, preferably from 5 µg to 17.5 mg, particularly preferably from 20 µg to 15 mg and very particularly preferably from 50 µg to 10 mg per g of the washing or cleaning agent,
and/or **in that** the protease in the washing or cleaning agent is encased by a substance that is impermeable to the protease at room temperature or in the absence of water.

4. The washing or cleaning agent according to claim 3, **characterized in that** it
(a) is present in solid form, in particular as a flowable powder having a bulk density of from 300 g/l to 1200 g/l, in particular from 500 g/l to 900 g/l, or
(b) is present in pasty or in liquid form, and/or
(c) is present as a single-component system, or
(d) is divided into a plurality of components.

5. The washing or cleaning agent according to one of claims 1 to 4,
**characterized in that**
it comprises one or more additional enzymes, in particular a protease, amylase, cellulase, hemicellulase, mannanase, pectinase, tannase, xylanase, xanthanase, β-glucosidase, carrageenase, perhydrolase, oxidase, oxidoreductase or a lipase and combinations thereof.

6. A method for cleaning textiles or hard surfaces, **characterized in that**, in at least one method step, a washing or cleaning agent according to one of claims 1 to 5 is used, in particular such that the protease is used in an amount of from 40 µg to 4 g, preferably from 50 µg to 3 g, particularly preferably from 100 µg to 2 g and very particularly preferably from 200 µg to 1 g.

7. The use of a washing or cleaning agent according to one of claims 1 to 5 for cleaning textiles or hard surfaces, in particular such that the protease contained in the washing or cleaning agent is used in an amount of from 40 µg to 4 g, preferably from 50 µg to 3 g, particularly preferably from 100 µg to 2 g and very particularly preferably from 200 µg to 1 g.

## Revendications

1. Produit lavant ou nettoyant, **caractérisé en ce qu'**il contient au moins une protéase, la protéase comprenant au moins une séquence d'acides aminés choisie dans le groupe constitué :
a) d'une séquence d'acides aminés identique à la séquence d'acides aminés indiquée dans la SEQ ID NO 3
b) d'une séquence d'acides aminés identique à la séquence d'acides aminés indiquée dans la SEQ ID NO 2.

2. Produit lavant ou nettoyant selon la revendication 1, **caractérisé en ce que** la protéase est présente naturellement dans un microorganisme, de préférence dans un champignon, dans une bactérie Gram-négative ou Gram-positive, dont préférentiellement dans une bactérie de l'espèce vibrio, en particulier *vibrio metschnikovii* et en particulier *vibrio metschnikovii* DSM ID 10-62,
ou **en ce qu'**elle est codée par un acide nucléique présent dans une cellule-hôte de numéro de dépôt DSM 23708.

3. Produit lavant ou nettoyant selon une des revendications 1 ou 2, **caractérisé en ce qu'**il contient la protéase dans une quantité de 2 µg à 20 mg, de préférence de 5 µg à 17,5 mg, de manière particulièrement préférée de 20 µg à 15 mg et de manière tout particulièrement préférée de 50 µg à 10 mg par g du produit lavant ou nettoyant,
et/ou **en ce que** la protéase dans le produit lavant ou nettoyant est enrobée d'une substance qui ne laisse pas passer la protéase à température ambiante ou en présence d'eau.

4. Produit lavant ou nettoyant selon la revendication 3, **caractérisé en ce qu'**il :
(a) se présente sous forme solide, en particulier sous forme de poudre fluide avec une masse volumique apparente de 300 g/l à 1200 g/l, en particulier de 500 g/l à 900 g/l, ou
(b) se présente sous forme pâteuse ou liquide, et/ou
(c) se présente sous la forme d'un système mono-composant, ou
(d) est réparti en plusieurs composants.

5. Produit lavant ou nettoyant selon l'une des revendications 1 à 4, **caractérisé en ce qu'**il comprend un ou plusieurs autres enzymes, en particulier une protéase, une amylase, une cellulase, une hémicellulase, une mannanase, une pectinase, une tannase, une xylanase, une xanthanase, une β-glucosidase, une carragénase, une perhydrolase, une oxydase, une oxydoréductase ou une lipase ainsi que leurs combinaisons.

6. Procédé de nettoyage de textiles ou de surfaces dures, **caractérisé en ce qu'**on utilise un produit lavant ou nettoyant selon l'une des revendications 1 à 5 dans au moins une étape de procédé, en particulier de sorte que la protéase soit utilisée dans une quantité de 40 µg à 4 g, de préférence de 50 µg à 3 g, particulièrement préférentiellement de 100 µg à 2 g et tout particulièrement préférentiellement de 200 µg à 1 g.

7. Utilisation d'un produit lavant ou nettoyants selon l'une des revendications 1 à 5 pour le nettoyage de textiles ou de surfaces dures, en particulier de sorte que la protéase contenue dans le produit lavant ou nettoyant soit utilisée dans une quantité de 40 µg à 4 g, de préférence de 50 µg à 3 g, de manière particulièrement préférée de 100 µg à 2 g et de manière tout particulièrement préférée de 200 µg à 1 g.
